Europäisches Patentamt

European Patent Office

Office européen des brevets

(11) Numéro de publication: **0 353 161 B1**

(12)

# FASCICULE DE BREVET EUROPEEN

(45) Date de publication de fascicule du brevet: **20.07.94**   (51) Int. Cl.⁵: **C11B 5/00**, C09K 15/04, A61K 7/48

(21) Numéro de dépôt: **89402156.7**

(22) Date de dépôt: **28.07.89**

(54) **Système anti-oxydant à base d'un ester d'ascorbyle stabilisé.**

(30) Priorité: **29.07.88 FR 8810295**

(43) Date de publication de la demande:
**31.01.90 Bulletin 90/05**

(45) Mention de la délivrance du brevet:
**20.07.94 Bulletin 94/29**

(84) Etats contractants désignés:
**BE CH DE ES FR GB IT LI NL**

(56) Documents cités:
GB-A- 1 081 233    GB-A- 1 193 027
GB-A- 1 370 303    US-A- 2 383 815
US-A- 2 462 663    US-A- 4 369 180

JOURNAL OF FOOD SCIENCE, vol. 51, no. 5, 1986, pages 1293-1296, Institute of Food Technologists, Chicago, US; J.R. MAHONEY, Jr. et al.: "Role of alpha-tocopherol, ascorbic acid, citric acid and EDTA as oxidants in model systems"

(73) Titulaire: **L'OREAL**
**14, Rue Royale**
**F-75008 Paris(FR)**

(72) Inventeur: **N'Guyen, Ouang Lan**
**45, avenue Alsace Lorraine**
**F-92160 Antony(FR)**
Inventeur: **Griat, Jacqueline**
**11, Ouai de la Baronnie**
**F-94480 Ablon(FR)**
Inventeur: **Millecamps, François**
**11, Square A. Renoir**
**F-75014 Paris(FR)**
Inventeur: **Lang, Gérard**
**44, avenue Lacour**
**F-95210 Saint Gratien(FR)**
Inventeur: **Forestier, Serge**
**16, Allée Ferdinand Buisson**
**F-77410 Claye Souilly(FR)**

(74) Mandataire: **Stalla-Bourdillon, Bernard et al**
**CABINET NONY & CIE**
**29, rue Cambacérès**
**F-75008 Paris (FR)**

EP 0 353 161 B1

## Description

La présente invention a pour objet un nouveau système anti-oxydant à base d'un ester d'ascorbyle stabilisé, contenant, en association au moins un tocophérol ou un mélange de tocophérols ou de l'acide caféique ou l'un de ses dérivés, au moins un agent complexant et au moins un polypeptide, l'utilisation d'un tel système anti-oxydant et des compositions à base de matières oléagineuses contenant un tel système, notamment des compositions cosmétiques.

On sait que les corps gras ont tendance à s'oxyder, même à température ambiante, et cette oxydation (ou rancissement) leur fait acquérir de nouvelles propriétés, notamment gustatives ou olfactives qui sont généralement considérées comme indésirables lorsque ces corps gras sont incorporés par example dans des compositions alimentaires ou dans des compositions cosmétiques.

On utilise donc couramment, dans les compositions contenant des corps gras, des agents protecteurs qui jouent en fait le rôle d'anti-oxydant.

Parmi les anti-oxydants connus, on utilise couramment l'acide ascorbique qui agit notamment par absorption directe d'oxygène. Toutefois, l'acide ascorbique est très peu soluble dans les corps gras et est donc difficilement utilisable pour protéger ceux-ci contre l'oxydation.

Afin de solubiliser la molécule d'acide ascorbique dans les matières grasses, on a proposé l'utilisation de divers esters d'ascorbyle tels que par exemple le stéarate, le palmitate ou le laurate d'ascorbyle; voir par exemple l'article de C.F. BOURGEOIS, "Revue 'Française des Corps Gras", n°9, pages 353-356 (Septembre 1981).

On sait qu'en dehors de leurs propriétés anti-oxydantes propres, les dérivés ascorbiques ont également la propriété d'améliorer l'activité d'agents anti-oxydants tels que les tocophérols ou l'acide caféique et ses esters, en favorisant la régénération de ces agents anti-oxydants; voir par exemple H.S. OLCOTT, "Oil Soap", 18, (1941), 77 et le brevet Us 2.462.663.

On a également proposé diverses améliorations de ces agents anti-oxydants binaires, du type dérivés ascorbiques + tocophérols ou dérivés ascorbiques + dèrivés caféiques, en prévoyant l'addition d'un troisième constituant améliorant encore les effets anti-oxydants. Parmi les troisièmes constituants de ces systèmes ternaires, on peut citer notamment l'acide p-amino-benzoïque (brevet US 2.462.663), des phospholipides (R.W. RIEMENSCHNEIDER et al., "Oil Soap" (1944), 47), des amines (KLAUI, "The Functional (Technical) Uses of Vitamins", ed. by M. STEIN, University of Nottingham Seminar Vitamins, London, England (1971), page 110), etc...

Le GB-A-1.193.027 décrit l'emploi en association avec un agent anti-oxydant tel qu'un tocophérol, d'un mélange de deux substances dites synergiques, à savoir la colamine et/ou un sel d'acide gras supérieur de colamine et un ester d'acide gras supérieur d'acide ascorbique.

Le GB-A-1.081.233 décrit la prolongation de la durée d'activité des anti-oxydants tels que la diphényl amine, à l'aide de l'EDTA neutralisé en association avec un mélange de diéthylène glycol et de polyéthylène glycol.

On a maintenant découvert qu'il est possible d'améliorer notablement les propriétés anti-oxydantes des esters d'ascorbyle en utilisant ces anti-oxydants conjointement avec au moins un tocophérol ou un mélange de tocophérols ou de l'acide caféique ou l'un de ses dérivés, au moins un agent complexant et au moins un polypeptide non thiolé. On observe un effet de synergie important.

La présente invention a donc pour objet un nouveau système anti-oxydant, exempt de composé réducteur, à base d'au moins un ester d'ascorbyle stabiiisé, caractérisé par le fait qu'il comprend au moins un tocophérol ou un mélange de tocophérols ou de l'acide caféique ou l'un de ses dérivés, au moins un agent complexant et au moins un polypeptide non thiolé.

L'ester d'ascorbyle est bien entendu un ester soluble dans les corps gras, et en particulier un ester d'un acide aliphatique ayant de 6 à 24 atomes de carbone tel que par exemple le stéarate, le palmitate ou le laurate d'ascorbyle.

Par "tocophérols", on entend non seulement l'$\alpha$-tocophérol mais également le $\beta$, $\gamma$ ou $\delta$ tocophérol ainsi que les mélanges de ceux-ci.

Les dérivés de l'acide caféique sont soit des esters soit des amides de cet acide.

Parmi les esters de l'acide caféique, on peut notamment citer les alkyl esters tels que les méthyl, éthyl ou butyl esters et le phytol ester.

Parmi les amides de l'acide caféique, on peut notamment citer les N-alkyl amides tels que le N-octyl amide.

On entend par agent complexant un composé qui est capable d'inhiber par chélation l'effet catalytique des métaux de transition à l'état libre dans le milieu.

2

Parmi les agents complexants utilisables, on citera notamment l'acide éthylènediamine tétracétique (EDTA), le sel pentasodique de l'acide diéthylènetriamine pentacétique (DTPA), le salicylate d'hexadécylamine (SHDA), l'acide citrique, l'acide tartrique et son sel de sodium, l'acide phytique, le dibenzyldithiocarbamate et les dérivés d'acide polyphosphonique ou un mélange de tels agents.

Parmi les dérivés d'acide polyphosphonique et leurs sels on peut notamment mentionner les acides alkylènediamino poly(méthylène phosphoni- ques) et notamment les produits vendus par la Société MONSANTO sous les dénominations de DEQUEST 2041 (acide éthylènediamine tétra (méthylène phosphonique)), DEQUEST 2046 (sel pentasodique du DEQUEST 2041), DEQUEST 2051 (acide hexaméthylènediamine tétra (méthylène phosphonique)), DEQUEST 2054 (sel hexapotassique du DEQUEST 2051), DEQUEST 2060S (acide diéthylènediamine penta (méthylène phosphonique)) et le DEQUEST 2066 (sel heptasodique du DEQUEST 2060S).

Outre au moins un des agents complexants énumérés ci-dessus, la composition selon l'invention peut également contenir un agent complexant secondaire comme le sorbitol.

Le polypeptide non thiolé du sytème anti-oxydant selon l'invention a un poids moléculaire moyen compris entre environ 1.000 et environ 100.000. Parmi les polypeptides que l'on peut utiliser, on peut en particulier mentionner les suivants:

(a) le polypeptide vendu sous la dénomination de "KERASOL" (polypeptide de la kératine soluble de poids moléculaire moyen d'environ 100.000) par la Société CRODA Chemicals Ltd,

(b) le polypeptide vendu sous la dénomination de "Polypeptide SF" (polypeptide de collagène animal partiellement neutralisé de poids moléculaire moyen d'environ 1.000) par la Société NAARDEN,

(c) le polypeptide vendu sous la dénomination de "Polypeptide LSN" (polypeptide de collagène animal sous forme de sel d'ammonium contenant environ 3% (max) de sel inorganique) par la Société NAARDEN, et

(d) le polypeptide vendu sous la dénomination de "LACTOLAN" (polypeptide obtenu à partir du lait frais de vache préalablement délipidé) par les LABORATOIRES SEROBIOLOGIQUES de NANCY.

L'ester d'ascorbyle est généralement présent dans le système anti-oxydant à quatre constituants, en une proportion comprise entre 5 et 70% en poids.

On a constaté de façon tout-à-fait surprenante que, dans de telles associations, l'activité anti-oxydante des tocophérols et de l'acide caféique, résultait d'un effet de synergie important avec l'ester d'ascorbyle, en présence du couple agent complexant - polypeptide non thiolé.

Selon l'invention, le système anti-oxydant est de préférence constitué de:

0,5 à 20% en poids tocophérol (s) ou d'acide caféique (ou d'un de ses esters ou amides)

5 à 70% en poids d'un ester d'ascorbyle

2 à 20% en poids d'un agent complexant

1 à 80% en poids d'un polypeptide non thiolé.

Le rapport molaire de l'ester d'ascorbyle au(x) tocophérol(s) ou à l'acide caféique ou un de ses esters ou amides doit être de préférence supérieur ou égal à 3.

L'invention a également pour objet des compositions contenant des corps gras, caractérisées par le fait qu'elles renferment au moins un système anti-oxydant tel que défini précédemment.

Les compositions de l'invention peuvent être notamment des compositions alimentaires (huiles comestibles, saindoux, beurre, margarine ou autres succédanés de beurre) ou des compositions cosmétiques.

Les corps gras présents dans les compositions cosmétiques de l'invention sont par exemple des corps gras d'origine animale tels que la cétine (blanc de baleine), la cire d'abeille, la lanoline, le perhydrosqualène, l'huile de tortue, etc...; des corps gras végétaux sous forme d'huiles, de graisses ou de cires tels que l'huile d'amande douce, l'huile d'avocat, l'huile d'olive,...; les huiles de coprah ou de palmiste hydrogénées, le beurre de cacao, la cire de Carnauba, la cire de Montana; ainsi que des huiles synthétiques constituées par des esters et/ou éthers de glycérol ou de glycol tels que par exemple ceux qui sont décrits dans les brevets français n° 75.24656, 75.24657 et 75.24658.

En plus des corps gras plus ou moins oxydables, les compositions cosmétiques peuvent contenir des produits sensibles à l'oxydation tels que par exemple de la vitamine F ou du $\beta$-carotène.

Les compositions cosmétiques selon l'invention se présentent sous la forme de solutions huileuses, d'émulsions, de produits solides, de lotions ou encore de micro-dispersions, de vésicules lipidiques ioniques ou non ioniques. Elles constituent notamment des laits pour les soins de la peau, des crèmes (crèmes pour le visage, pour les mains, pour le corps, crèmes anti-solaires, crèmes démaquillantes, crèmes fonds de teint), des fonds de teint fluides, des laits démaquillants, des laits anti-solaires, des huiles pour le bain, des rouges à lèvres, des fards à paupières, des sticks déodorants, etc...

Selon une forme de réalisation préférée, les compositions cosmétiques se présentent sous forme de crèmes destinées à la protection de l'oxydation des lipides de la peau.

3

Dans les compositions cosmétiques selon l'invention, le système anti-oxydant tel que défini ci-dessus est généralement présent de sorte que l'on ait les proportions suivantes par rapport au poids total de la composition:

| | |
|---|---|
| - Tocophérol(s) ou acide caféique (ou un de ses esters ou amides) | 0,05 à 0,5% en poids |
| - Ester d'ascorbyle | 0,45 à 1,6% en poids |
| - Agent complexant | 0,2 à 0,5% en poids |
| - Polypeptide (en matière active) | 0,05 à 8% en poids |

Les compositions de l'invention peuvent en outre contenir des composés actifs ou des ingrédients utilisés de façon usuelle dans les compositions mentionnées ci-dessus, tels que des agents tensio-actifs, des colorants, des parfums, des produits astringents, des produits absorbant l'ultra-violet, des solvants organiques, de l'eau, etc...

Ces compositions sont préparées selon les méthodes usuelles.

On va maintenant donner à titre d'illustration plusieurs exemples de systèmes anti-oxydants selon l'invention ainsi que des exemples de compositions cosmétiques contenant de tels systèmes anti-oxydants.

EXEMPLE 1

| | |
|---|---|
| - Tocophérols (mélange $\alpha, \beta$ , $\gamma$ et $\delta$ | 5% |
| - Palmitate d'ascorbyle | 65% |
| - EDTA | 5% |
| - Polypeptide "Polypeptide SF" (matière active) | 25% |

EXEMPLE 2

| | |
|---|---|
| - Acide caféique | 20% |
| - Palmitate d'ascorbyle | 50% |
| - Acide citrique | 10% |
| - Sorbitol | 10% |
| - Polypeptide "KERASOL" (matière active) | 10% |

EXEMPLE 3

| | |
|---|---|
| - Ester méthylique de l'acide caféique | 13% |
| - Palmitate d'ascorbyle | 10% |
| - DPTA | 12% |
| - Polypeptide "Polypeptide LSN" (matière active) | 65% |

4

EXEMPLES DE COMPOSITIONS COSMETIQUES

I - Baume anhydre

| | |
|---|---|
| - Huile de karité | 60% |
| - Huile de tournesol | 20% |
| - Vitamine F | 2% |
| - Lécithine de soja | 4,9% |
| - Tocophérols | 0,45% |
| - Palmitate d'ascorbyle | 1,6% |
| - Acide citrique | 0,10% |
| - EDTA | 2% |
| - Polypeptide "LACTOLAN" (matière active) | 1% |
| - Vaseline q.s.p | 100% en poids |

II - Crème de soin émulsion huile-dans-l'eau

| | |
|---|---|
| - Monostéarate de sorbitan à 20 moles d'oxyde d'éthylène (Tween 60) | 1% |
| - Stéarate de glycérol | 2% |
| - Acide stéarique | 1,4% |
| - Triéthanolamine | 0,7% |
| - Alcool cétylique | 0,5% |
| - Huile de tournesol | 15% |
| - Vitamine F | 2% |
| - Acide ascorbique | 1% |
| - Lécithine de soja | 0,6% |
| - Huile de vaseline | 2,4% |
| - Acide caféique | 0,1% |
| - Palmitate d'ascorbyle | 0,6% |
| - DTPA | 0,5% |
| - Polypeptide "Polypeptide LSN" (matière active) | 7% |
| - Polymère carboxyvinylique vendu sous le nom de "Carbopol 940" par la Société Goodrich | 0,2% |
| - Triéthanolamine | 0,2% |
| - Parfum | 0,8% |
| - Conservateur (Parahydroxybenzoate de méthyle) | 0,25% |
| - Eau q.s.p | 100% en poids |

III - Gel de soin pour le visage sous forme de dispersion vésiculaire

1ère phase : On prépare une dispersion aqueuse de sphérules lipidiques selon le procédé décrit dans le brevet francais n° 75 20456 (2.315.991) à partir des substances suivantes:

- lipide amphiphile non ionique de formule générale :

$$R \longleftarrow (OCH_2 - CH)_n \longrightarrow OH$$
$$| $$
$$CH_2OH$$

dans laquelle R est un radical hexadétyle et n a une valeur

statistique moyenne égale à 3 ......................     4   g

     – Cholestérol ..............................     4   g

     – Parahydroxybenzoate de méthyle .............    0,3 g

     – Glycérine .................................     3   g

     – Eau déminéralisée .........................   42,5 g

2ème phase : On ajoute à la dispersion de sphérules obtenues dans la 1ère phase, les substances suivantes :

| | |
|---|---|
| - Parfum | 0,4 g |
| - Huile de Maïs | 18, g |
| - Tocophérols (mélange $\alpha,\beta,\gamma$ et $\delta$) | 0,5 g |
| - Palmitate d'ascorbyle | 1, g |
| - EDTA | 0,3 g |
| - Polypeptide "Polypeptide SF" (matière active) | 5 g |
| - Polymère carboxyvinylique vendu sous le nom de "Carbopol 940" par la Société GOODRICH | 0,4 g |
| - Triéthanolamine | 0,4 g |
| - Eau déminéralisée | 20,2 g |

## Revendications

1. Système anti-oxydant à base d'au moins un ester d'ascorbyle stabilisé, caractérisé par le fait qu'il comprend au moins un tocophérol ou un mélange de tocophérols ou de l'acide caféique ou l'un de ses dérivés, au moins un agent complexant et au moins un polypeptide non thiolé.

2. Système anti-oxydant selon la revendication 1, caractérisé par le fait que l'ester d'ascorbyle est un ester d'acide aliphatique ayant de 6 à 24 atomes de carbone tel que le stéarate, le palmitate ou le laurate d'ascorbyle.

3. Système anti-oxydant selon la revendication 1, caractérisé par le fait que les dérivés d'acide caféique sont des alkyl esters tels que les méthyl, éthyl ou butyl esters et le phytol ester ou des N-alkyl amides tels que le N-octylamide.

4. Système anti-oxydant selon la revendication 1, caractérisé par le fait que l'agent complexant est l'acide éthylènediamine tétracétique, le sel pentasodique de l'acide diéthylènetriamine pentacétique, le salicylate d'hexadécylamine, l'acide citrique, l'acide tartrique, le tartrate de sodium, l'acide phytique, le dibenzyldithiocarbamate ou un dérivé d'acide polyphosphonique ou un mélange de tels agents.

5. Système anti-oxydant selon l'une quelconque des revendications précédentes, caractérisé par le fait qu'il contient en outre un agent complexant secondaire tel que le sorbitol.

6. Système anti-oxydant selon la revendication 1, caractérisé par le fait que le polypeptide non thiolé a un poids moléculaire compris entre 1.000 et 100.000.

7. Système anti-oxydant selon la revendication 1 ou 2, caractérisé par le fait que l'ester d'ascorbyle est présent à raison de 5 à 70% en poids.

8. Système anti-oxydant selon l'une quelconque des revendications précédentes, caractérisé par le fait que l'agent complexant est présent à raison de 2 à 20% en poids et que le polypeptide non thiolé est présent à raison de 1 à 80% en poids.

9. Système anti-oxydant selon l'une quelconque des revendications précédentes, caractérisé par le fait qu'il est constitué de:
   0,5 à 20% en poids de tocophérol(s) ou d'acide caféique
(ou d'un de ses esters ou amides)
   5 à 70% en poids d'un ester d'ascorbyle
   2 à 20% en poids d'un agent complexant
   1 à 80% en poids d'un polypeptide non thiolé.

10. Système anti-oxydant selon l'une quelconque des revendications précédentes, caractérisé par le fait que le rapport molaire de l'ester d'ascorbyle au(x) tocophérol(s) ou à l'acide caféique ou à l'un de ses dérivés est supérieur ou égal à 3.

11. Composition contenant des corps gras, caractérisée par le fait qu'elle contient un système anti-oxydant selon l'une quelconque des revendications 1 à 10.

12. Composition cosmétique, caractérisée par le fait qu'elle contient un système anti-oxydant selon l'une quelconque des revendications 1 à 10, les proportions des constituants du système anti-oxydant par rapport au poids total de la composition étant de:

| | |
|---|---|
| - Tocophérol(s) ou acide caféique (ou un de ses esters ou amides) | 0,05 à 0,5% en poids |
| - Ester d'ascorbyle | 0,45 à 1,6% en poids |
| - Agent complexant | 0,2 à 0,5% en poids |
| - Polypeptide (en matière active) | 0,05 à 8% en poids |

13. Composition cosmétique selon la revendication 12, caractérisée par le fait que l'ester d'ascorbyle est le palmitate d'ascorbyle.

14. Composition cosmétique selon la revendication 12, caractérisée par le fait que l'agent complexant est l'acide éthylènediamine tétracétique, l'acide citrique, le sel pentasodique de l'acide diéthylènetriamine pentacétique, le dibenzyldithiocarbamate ou un dérivé d'acide polyphosphonique ou un mélange de tels agents.

15. Composition cosmétique selon l'une quelconque des revendications 12 à 14, caractérisée par le fait qu'elle se présente sous forme d'une crème destinée à la protection de l'oxydation des lipides de la peau.

**Claims**

1. Antioxidant system based on at least one stabilized ascorbyl ester, characterized in that it comprises at least one tocopherol or a mixture of tocopherols or caffeic acid or one of its derivatives, at least one complexing agent and at least one unthiolated polypeptide.

2. Antioxidant system according to Claim 1, characterized in that the ascorbyl ester is an aliphatic acid ester having from 6 to 24 carbon atoms, such as ascorbyl stearate, palmitate or laurate.

3. Antioxidant system according to Claim 1, characterized in that the caffeic acid derivatives are alkyl esters such as the methyl, ethyl or butyl esters and the phytol ester or N-alkylamides such as the N-octylamide.

4. Antioxidant system according to Claim 1, characterized in that the complexing agent is ethylenediaminetetraacetic acid, diethylenetriaminepentaacetic acid pentasodium salt, hexadecylamine salicylate, citric acid, tartaric acid, sodium tartrate, phytic acid, dibenzyldithiocarbamate or a polyphosphonic acid derivative, or a mixture of such agents.

5. Antioxidant system according to any one of the preceding claims, characterized in that it contains, in addition, a secondary complexing agent such as sorbitol.

6. Antioxidant system according to Claim 1, characterized in that the unthiolated polypeptide has a molecular weight of between 1,000 and 100,000.

7. Antioxidant system according to Claim 1 or 2, characterized in that the ascorbyl ester is present in the proportion of 5 to 70 % by weight.

8. Antioxidant system according to any one of the preceding claims, characterized in that the complexing agent is present in the proportion of 2 to 20 % by weight and in that the unthiolated polypeptide is present in the proportion of 1 to 80 % by weight.

9. Antioxidant system according to any one of the preceding claims, characterized in that it consists of:
   0.5 to 20 % by weight of tocopherol (s) or of caffeic acid (or of one of its ester or amides)
   5 to 70 % by weight of an ascorbyl ester
   2 to 20 % by weight of a complexing agent
   1 to 80 % by weight of an unthiolated polypeptide

10. Antioxidant system according to any one of the preceding claims, characterized in that the mol ratio of the ascorbyl ester to the tocopherol(s) or to caffeic acid or to one of its derivatives is greater than or equal to 3.

11. Composition containing fats, characterized in that it contains an antioxidant system according to any one of Claims 1 to 10.

12. Cosmetic composition, characterized in that it contains an antioxidant system according to any one of Claims 1 to 10, the proportions of the constituents of the antioxidant system relative to the total weight of the composition being:

| - Tocopherol(s) or caffeic acid (or one of its esters or amides) | 0.05 to 0.5 % by weight |
| - Ascorbyl ester | 0.45 to 1.6 % by weight |
| - Complexing agent | 0.2 to 0.5 % by weight |
| - Polypeptide (as active substance) | 0.05 to 8 % by weight |

13. Cosmetic composition according to Claim 12, characterized in that the ascorbyl ester is ascorbyl palmitate.

14. Cosmetic composition according to Claim 12, characterized in that the complexing agent is ethylenediaminetetraacetic acid, citric acid, diethylenetriaminepentaacetic acid pentasodium salt, dibenzyldithiocarbamate or a polyphosphonic acid derivative, or a mixture of such agents.

15. Cosmetic composition according to any one of Claims 12 to 14, characterized in that it is presented in the form of a cream intended for protection of the lipids of the skin against oxidation.

**Patentansprüche**

1. Antioxidationssystem auf Grundlage mindestens eines stabilisierten Ascorbinsäureesters, **dadurch gekennzeichnet,** daß es mindestens ein Tocopherol oder eine Mischung von Tocopherolen oder Kaffeesäure oder ein Derivat davon, mindestens einem Komplexierungsmittel und mindestens einem Polypeptid ohne Thiolfunktionen enthält.

2. Antioxidationssystem nach Anspruch 1, **dadurch gekennzeichnet,** daß der Ascorbinsäureester ein Ester einer aliphatischen Säure mit 6 bis 24 Kohlenstoffatomen, wie Ascorbinsäurestearat, Ascorbinsäurepalmitat oder Ascorbinsäurelaurat ist.

3. Antioxidationssystem nach Anspruch 1, **dadurch gekennzeichnet,** daß die Derivate der Kaffeesäure Alkylester, wie der Methyl-, Ethyl-, oder Butylester und der Phytolester, oder die N-Alkylamide, wie das N-Octylamid sind.

4. Antioxidationssystem nach Anspruch 1, **dadurch gekennzeichnet,** daß das Komplexierungsmittel Ethylendiamintetra-Essigsäure, das Pentanatriumsalz der Diethylentriaminpenta-Essigsäure, das Hexadecylaminsalicylat, Zitronensäure, Weinsäure, Natriumtartrat, Phytolsäure, Dibenzyldithiocarbamat oder ein Derivat der Polyphosphonsäure oder eine Mischung dieser Wirkstoffe ist.

5. Antioxidationssystem nach einem der vorhergehenden Ansprüche, **dadurch gekennzeichnet,** daß es außerdem ein sekundäres Komplexierungsmittel, wie Sorbit, enthält.

6. Antioxidationssystem nach Anspruch 1 **dadurch gekennzeichnet,** daß das Polypeptid ohne Thiolfunktion ein Molekulargewicht zwischen 1 000 und 100 000 besitzt.

7. Antioxidationssystem nach Anspruch 1 oder 2, **dadurch gekennzeichnet,** daß der Ascorbinsäureester in einer Menge von 5 bis 70 Gew.% vorhanden ist.

8. Antioxidationssytem nach einem der vorhergehenden Ansprüche, **dadurch gekennzeichnet,** daß das Komplexierungsmittel in einer Menge von 2 bis 20 Gew.% und das Polypeptid ohne Thiofunktion in einer Menge von 1 bis 80 Gew.% vorhanden ist.

9. Antioxidationssystem nach einem der vorhergehenden Ansprüche, **dadurch gekennzeichnet,** daß es enthält:
0,5 bis 20 Gew.% Tocopherol(2) oder kaffeesäure (oder einen Ester oder ein Amid),
5 bis 70 Gew.% Ascorbinsaüreester,
2 bis 20 Gew.% Komplexierungsmittel,
1 bis 80 Gew.% Polypeptid ohne Thiolfunktion.

10. Antioxidationssystem nach einem der vorhergehenden Ansprüche, **dadurch gekennzeichnet,** daß das molare Verhältnis des Ascorbinsäureesters zum (zu den) Tocopherol(en) oder der Kaffeesäure oder zu einem deren Derivate gleich oder größer als 3 ist.

11. Fetthaltige Zusammensetzung, **dadurch gekennzeichnet,** daß sie ein Antioxidationssystem nach einem der Ansprüche 1 bis 10 enthält.

12. Kosmetische Zusammensetzung, **dadurch gekennzeichnet,** daß sie ein Antioxidationssystem nach einem der Ansprüche 1 bis 10 enthält, wobei die Anteile des Antioxidationssystems, bezogen auf das Gesamtgewicht der Zusammensetzung, die folgenden sind:

| | |
|---|---|
| Tocopherol(e) oder Kaffeesäure (oder einen Ester oder ein Amid) | 0,05 bis 0,5 Gew.% |
| Ascorbinsäureester | 0,45 bis 1,6 Gew.% |
| Komplexierungsmittel | 0,2 bis 0,5 Gew.% |
| Polypeptid (Wirkstoff) | 0,05 bis 8 Gew.% |

13. Kosmetische Zusammensetzung nach Anspruch 12, **dadurch gekennzeichnet,** daß der Ascorbinsäureester Ascorbinsäurepalmitat ist.

14. Kosmetische Zusammensetzung nach Anspruch 12, **dadurch gekennzeichnet,** daß das Komplexierungsmittel Ethylendiamintetra-Essigsäure, Zitronensäure, das Pentanatriumsalz der Diethylentriaminpenta-Essigsäure, Dibenzyldithiocarbamat oder ein Polyphosphonsäurederivat oder eine Mischung dieser Stoffe ist.

15. Kosmetische Zusammensetzung nach einem der Ansprüche 12 bis 14, **dadurch gekennzeichnet,** daß sie in Form einer Creme vorliegt, die zum Schutz der Lipide der Haut vor Oxidation bestimmt ist.